# EUROPÄISCHE PATENTANMELDUNG

(11) **EP 2 591 791 A1**
(43) Veröffentlichungstag der Anmeldung: **15.05.2013**
(21) Anmeldenummer: 11188313.8
(22) Anmeldetag: 08.11.2011
(51) Int. Cl.: A61K 36/88, A61K 8/00, A61K 9/00, A61P 17/02

(54) **Bulbine-frutescens-Pflanzensaft**

(71) Anmelder: Cremer Oleo Gmbh & Co.Kg, 20095 Hamburg (DE)
(72) Erfinder: Spangenberg, Tilman, 20095 Hamburg (DE); Coetsee, Wilhelm, Cape Town (ZA)
(74) Vertreter: Richter Werdermann Gerbaulet Hofmann

(57) **Zusammenfassung**

Die Erfindung betrifft Bulbine-frutescens-Pflanzensaft, erhältlich durch Verarbeitung der Blätter der Bulbine fructescens in einem speziellen Pressverfahren und anschließendem Pasteurisieren. Der Bulbine-frutescens-Pflanzensaft findet im therapeutischen oder kosmetischen Bereich Verwendung und zeigt eine besonders gute Wirkung bei Erkrankungen der Haut- und Schleimhaut, insbesondere Infektionen und Entzündungen, wie Pilzerkrankungen, bei der Vorbeugung und/oder Heilung von Verletzungen der Haut und Schleimhaut, insbesondere bei (Brand-)Wunden, als Desinfektionsmittel, als Mittel zur Stärkung der Immunabwehr, zur Körperpflege, Mundpflege, in Deodorants und als Anti-Ageing-Produkt. Die Wirkung ist zum Teil handelsüblichen Produkten weit überlegen, wobei die natürliche Mikroflora der Haut und der Schleimhäute nicht geschädigt wird, sondern vielmehr intakt erhalten bleibt. Außerdem wird das Erscheinungsbild der Haut deutlich verbessert.

## Beschreibung

Die Erfindung betrifft Bulbine-frutescens-Pflanzensaft, ein Verfahren zu dessen Herstellung, dessen Verwendung im medizinischen und kosmetischen Bereich sowie Zusammensetzungen, umfassend den erfindungsgemäßen Bulbine frutescens-Pflanzensaft.

Die Bulbine frutescens (L.), die zur Familie der Asphodeloideae (Affodillgewächse) gehört, ist auch unter den Bezeichnungen "Rankkopieva", "Ibhucu" oder "Ithethe elimpofu" bekannt. Der deutsche Name lautet Stelzen-Bulbine. Die Pflanze ist in ganz Südafrika weit verbreitet; wobei mehr als 40 Unterarten bekannt sind. Sie wächst bevorzugt in sandigen Böden. Die Pflanze hat einen Wurzelstock mit zahlreichen drahtartigen Wurzeln, die Blätter sind 15 cm lang und 4 bis 8 mm dick, von hellgrüner Farbe, unbehaart und fleischig. Von April bis August trägt die Pflanze gelbe, orangefarbene oder weiße Blüten in dichten länglichen Blütentrauben mit einer Länge bis zu 30 cm und bärtigen Staubblättern.

Bekannt ist es, durch Auspressen aus den Blättern einen geleeartigen Saft zu gewinnen, der als "Allheilmittel" beispielsweise gegen (brennende) Wunden, Schürfwunden, wunde Stellen, Hautausschlag, Ekzeme und Ringelflechte verwendet wird. Der Saft wird aus einem frisch gepflückten Blatt gepreßt und auf den betroffenen Hautbereich aufgetragen. Es ist außerdem ein Tee-Aufguss aus den frischen Blätter bekannt, der gegen Erkältung, Husten, Arthritis, Durchfall, Blasenentzündungen, Infektionen der Harnwege und bei einigen sexuell übertragbaren Krankheiten helfen soll. Die Wirksamkeit ist bisher nicht klinisch erwiesen und es sind auch keinerlei Fachberichte hierüber veröffentlicht worden.

Zur chemischen Zusammensetzung sind jedoch einige Informationen verfügbar. So berichtet Van Staden (Phytochemistry 35, 685 (1994)), dass Knipholon in Bulbine latifolia und Bulbine frutescens vorhanden ist. Knipholon ist abgeleitet von 1,8-Dihydroxyanthrachinon und trägt in der 3-Stellung eine Methylgruppe und in der 4-Stellung eine 2,6-Dihydroxy-4-methoxy-3-acetylphenylgruppe, die auch als 2-Acetylphloroglucinmethylether bezeichnet wird; Isoknipholon weist die 2-Acetylphloroglucinmethylethergruppe in der 9-Stellung auf. 1,8-Dihydroxy-3-methylanthrachinon ist besser bekannt als Chrysophanol.

Das Vorkommen von Knipholon und seinen Derivaten ist praktisch auf die Bulbinearten beschränkt, obwohl Anthrachinone in der Natur weit verbreitet sind. Knipholon wurde nur in Kniphofia-Arten gefunden, wie beispielsweise von Dagne und Steglich (Phytochemistry 23, 1729 (1984) und Bull. Chem. Soc. Ethiop. 32, 1 (1987)) beschrieben. Leistner et al. (Chem. Pharm. Bull. 52, 1262 (2004)) haben Knipholon-Derivate in Kniphofia foliosa und Kniphofia tuckii, die in Äthiopien beheimatet sind, mit Schwerpunkt auf der Glucose und dem Disaccharid Gentiobiose als glucosidischem Teil dieser wasserlöslichen Anthrachinone, beschrieben.

Seit dem ersten Bericht über Knipholon in Vertretern der Familie der Asphodeloideae wurde über eine Anzahl weiterer arylsubstituierter Chrysophanol-Derivate, entweder als Glycoside oder als Aglycone, berichtet. Bringmann et al. (J. Nat. Prod. 65, 1117 (2002)) beschrieben Gaborochinon A und B, 4`-*O-*Dimethylknipholon-4'-O-β-d-glucopyranosid und Phenylanthrachinone aus den Wurzeln der Bulbine frutescens. Einige dieser Verbindungen zeigten *in vitro* moderate bis gute antiplasmodiale und antitrypanosomale Wirkungen. Bringmann et al. (2007) fanden außerdem in den Wurzeln der Bulbine frutescens dimere Knipholon-Derivate (Joziknipholon A und B). *In vitro* zeigten diese Joziknipholone Wirkung gegen den chloroquinresistenten Strang K1 des Malariaerregers Plasmodium falciparum und moderate Wirkung gegen L5178Y-Leukämie-Lymphom-Mäusezellen.

Rabe und Van Staden (J. Ethnopharmacology 56, 81 (1997)) untersuchten die *In-vitro*-Wirkung getrockneter Extrakte aus den Blättern der Bulbine frutescens auf ihre mikrobiologische Wirkung gegen Staphylococcus aureus, Staphylococcus epidermidis and Bacillis subtilis; die Extrakte erwiesen sich jedoch als unwirksam. Van Staden und Drewes testeten Knipholon *in vitro* auf antimikrobielle Wirkung gegen Escherichia coli, Pseudomonas aeruginosa, Staphylococcus aureus, Bacillus subtilis, Micrococcus luteus und Candida albicans; dieses erwies sich jedoch ebenfalls als nicht wirksam. Berichte zur Bioverfügbarkeit von Bulbine frutescens wurden bislang nicht veröffentlicht.

Substituierte 1,8-Dihydroxyanthrachinone sind auch in verschiedenen Aloearten, wie Aloe barbadensis, Aloe ferox (welche ebenfalls zur Familie der Asphodeloideae gehören) und in Rhabarberarten, wie Rheum rhabarbarum und Rheum palmatum (Chinesischer Rhabarber), zu finden. Den Anthrachinonen in den Aloe- und Rhabarberarten fehlt die in Knipholon vorhandene 2-Acetylchloroglucinmethylethergruppe; die Methylgruppe in der 3-Stellung kann auch in oxidierter Form vorhanden sein, entweder als Hydroxymethylgruppe, als Aldehyd oder als Säure. Die Anthrachinone in den Aloe- und Rhabarberarten sind als Glycoside vorhanden; die Zuckerreste können Pentosen und/oder Hexosen sein. Diese sind gut wasserlöslich, aber nach Alterung dieser Extrakte oder übermäßiger Erhitzung kann Hydrolyse auftreten und die Anthrachinone werden in Wasser unlöslich.

Die in Bulbinearten vorhandenen Anthrachinone zeigen ein völlig anderes physiologisches Potential als die Anthrachinone, die aus Aloe- und Rhabarberarten stammen. Die Anthrachinone aus Aloe- und Rhabarberarten zeigen oft abführende Eigenschaften. Die grundlegende Verbindung 1,8-Dihydroxyanthrachinon (Danthron®) wurde 1987 weltweit aus dem Handel genommen, da begründeter Verdacht bestand, dass diese Verbindung für den Menschen karzinogen ist. Toxische Nebenwirkungen, wie bei Aloe-/Rhabarber-Anthrachinonen, treten bei Knipholon-Anthrachinonen im Wesentlichen jedoch nicht auf. Hersteller von Aloegelen sind demnach stark bemüht, den Anthrachinon-Anteil so weit wie möglich zu reduzieren, da bei Aloegelen hauptsächlich der Polysaccharidanteil von Bedeutung ist.

Weiterhin ist aus der US 2010/0062085 A1 von A. D. Widgerow sowie aus weiteren US-Patentschriften von Widgerow und Chait bekannt, aus einem Extrakt der Bulbine frutescens ein Gel in Kombination mit Centella asiatica (auch bekannt als Tigergras, Indischer Wassernabel oder Gotu Cola) und Panthenol, der Vorstufe von Pantothensäure, zur Behandlung von Narben und Akne einzusetzen. Centella asiatica ist eine bekannte Quelle für eine Vielzahl pentacyclischer Triterpenoide, wie Asiaticosid, Centellosid und Madecassosid, nebst zahlreichen anderen chemischen Verbindungen. Die Offenbarung der US 2010/0062085 A1 erscheint jedoch äußerst fragwürdig, da der Beitrag von Bulbine frutescens bei der Verhinderung der Vernarbung klinisch nicht nachgewiesen ist.

Es besteht demnach ein Bedarf weitere Mittel für unterschiedliche therapeutische und andere Zweck bereitzustellen, die gegenüber bekannten Verbindungen über ein verbessertes und nachweisbares Wirkungsspektrum verfügen, in verschiedenen Bereichen zum Einsatz kommen können sowie in einfacher Weise erhältlich und anwendbar sind.

Die vorliegende Erfindung betrifft Bulbine-frutescens-Pflanzensaft, ein Verfahren zu dessen Herstellung sowie die Verwendung des Bulbine-frutescens-Pflanzensafts im medizinischen oder kosmetischen Bereich. Gegenstand der Erfindung ist auch eine pharmazeutische oder kosmetische Zusammensetzung, umfassend den Pflanzensaft. Die Verwendung im medizinischen Bereich betrifft im Wesentlichen die Behandlung von Haut- oder Schleimhauterkrankungen, insbesondere solche mit mikrobiologischer Ursache, beispielsweise hervorgerufen durch opportunistische Mikroorganismen oder durch hypo-/hyperaktive Enzyme. Im kosmetischen Bereich kann der Pflanzensaft beispielsweise zur Körperpflege eingesetzt werden. Der Pflanzensaft der Erfindung dient auch zur Stärkung des Immunsystems und ist daher zur Behandlung von immungeschwächten Personen besonders gut geeignet.

Die Erfindung bezieht sich daher auf Bulbine-frutescens-Pflanzensaft, erhältlich durch die Schritte:
(1) Waschen der Blätter der Bulbine frutescens mit Wasser und gegebenenfalls Zerkleinern der Blätter;
(2) Auspressen der Blätter unter Erhalt eines Presskuchens und von Pflanzensaft;
(3) Auflockern des erhaltenen Presskuchens;
(4) Auspressen des aufgelockerten Presskuchens unter Erhalt von weiterem Pflanzensaft;
(5) Zusammengeben des Pflanzensafts aus Schritt (2) und (4) und
(6) Pasteurisieren des aus Schritt (5) erhaltenen Pflanzensafts.

Nachfolgend werden die einzelnen Verfahrensschritte des erfindungsgemäßen Verfahrens zur Gewinnung des Bulbine-frutescens-Pflanzensafts detailliert beschrieben:
Die Blätter der Bulbine frutescens können das ganze Jahr hindurch geerntet werden, wobei in der Regel mindestens zwei Mal geerntet wird. Besonders bevorzugt wird in den frühen Morgenstunden geerntet. Es ist zweckmäßig, die Blätter direkt nach dem Ernten schnell weiterzuverarbeiten, so dass die Blätter möglichst frisch eingesetzt werden. Im Rahmen der Erfindung werden unter "Blätter" sämtliche geernteten und verwendbaren Pflanzenteile der Bulbine frutescens verstanden, die sich jedoch hauptsächlich aus den Blättern zusammensetzen.

Bevor die Verarbeitung der Blätter beginnt, wird in der Regel eine Qualitätskontrolle durchgeführt, wobei zunächst die Abstammung bzw. Artenreinheit der Pflanzen überprüft wird, d.h. ob es sich tatsächlich um die Blätter der Bulbine frutescens handelt. Es wird auch der Zustand und die Sauberkeit des Pflanzenmaterials geprüft, um Verunreinigungen und Fremdstoffe bereits im Vorfeld entsprechend aussortieren zu können.

Dann werden die Blätter mit Wasser gewaschen (Verfahrensschritt (1)), um Verschmutzungen, wie Erde, zu entfernen. Hierzu werden die Pflanzenteile, bevorzugt hauptsächlich die Blätter, in einen Waschbereich überführt. Das Waschen ist nicht besonders beschränkt. Es kann chargenweise in einem Behälter, beispielsweise einer Wanne oder einem Trog, oder auch kontinuierlich in einem Kanal mit fließendem Wasser gewaschen werden. Das Wasser ist bevorzugt besonders sauberes Wasser, beispielsweise entkeimtes Wasser, wie unter Verwendung von UV-Strahlung entkeimtes Wasser, oder desinfiziertes Wasser oder Reinstwasser. Nach dem Waschen werden die gewaschenen Pflanzenteile bevorzugt getrocknet. Dies erfolgt bevorzugt durch Verteilen auf Gestellen oder Gitterrosten, auf denen die Pflanzenteile zum Abtropfen und Trocknen aufgehängt oder aufgelegt werden. Das Waschen und Trocknen kann auch in anderer Weise erfolgen, beispielsweise durch Aufsprühen von Wasser und anschließendes Trockenblasen mit Luft.

Anschließend wird der Pflanzensaft mit Hilfe einer geeigneten Vorrichtung aus den Blättern heraus gepresst (Verfahrensschritte (2) bis (4)). Es kann eine dem Fachmann im Stand der Technik bekannte Presse eingesetzt werden. Beispielsweise wird eine Korbpresse eingesetzt. Es kann aber auch jede andere Art an bekannter Presse zum Einsatz kommen. Es können auch mehrere verschiedene Pressen für die einzelnen Pressvorgänge eingesetzt werden.

Vor dem Pressen können die Blätter gegebenenfalls zerkleinert werden, um das Auspressen zu erleichtern.

Erfindungsgemäß wird zunächst ein Auspressen der Blätter durchgeführt (Verfahrensschritt (2)), wonach das gepresste Blättermaterial aufgelockert wird (Verfahrensschritt (3)) und sich ein erneutes Auspressen (Verfahrensschritt (4)) anschließt.

Die beiden Auspressvorgänge der Verfahrensschritte (2) und(4) werden bevorzugt jeweils bei einem Druck im Bereich von etwa 200 bis 400 kPa, bevorzugt etwa 250 bis 350 kPa, noch bevorzugter 270 bis 320 kPa, insbesondere bei einem Druck von etwa 300 kPa durchgeführt. Die Presszeit beträgt bevorzugt einige Minuten bis etwa 2 Stunden, besonders bevorzugt etwa 10 Minuten bis etwa 1,5 Stunden, noch bevorzugter etwa 30 Minuten bis 1 Stunde.

Nach dem Auspressen gemäß Verfahrensschritt (2) wird der erhaltene Presskuchen in Verfahrensschritt (3) aufgelockert. Dies erfolgt beispielsweise, indem der Presskuchen aus der Presse entfernt, aufgeschüttelt und wieder in die Presse gegeben wird.

In einer besonders bevorzugten Ausführungsform der vorliegenden Erfindung wird das Auspressen der Blätter in einem dreistufigen Press-Verfahren, bevorzugt ohne weitere Zwischenschritte, durchgeführt, umfassend
(I) Pressen der Blätter bei einem Druck im Bereich von etwa 10 bis 100 kPa, bevorzugt etwa 20 bis 80 kPa, bevorzugter etwa 30 bis 70 kPa, insbesondere etwa 50 kPa für eine vorbestimmte Zeitdauer;
(II) Nachfüllen der aus Verfahrensschritt (1) erhaltenen gewaschenen, gegebenenfalls zerkleinerten Blätter und dann Pressen bei einem Druck im Bereich von etwa 50 bis 200 kPa, bevorzugter etwa 70 bis 180 kPa, noch bevorzugter 80 bis 150 kPa, insbesondere etwa 100 kPa für eine vorbestimmte Zeitdauer und
(III) Nachfüllen der aus Verfahrensschritt (1) erhaltenen gewaschenen, gegebenenfalls zerkleinerten Blätter und dann Pressen bei einem Druck im Bereich von etwa 200 bis 400 kPa, bevorzugter etwa 250 bis 350 kPa, noch bevorzugter 270 bis 320 kPa, insbesondere etwa 300 kPa für eine vorbestimmte Zeitdauer.

In Pressstufe (I) werden die gewaschenen und gegebenenfalls zerkleinerten Blätter der Bulbine frutescens bei etwa 10 bis 100 kPa, bevorzugt etwa 20 bis 80 kPa, bevorzugter etwa 30 bis 70 kPa, insbesondere etwa 50 kPa für eine vorbestimmte Zeit unter Erhalt von Pflanzensaft (Pflanzensaft I) ausgepresst. Im Anschluss daran wird der Pressvorgang unterbrochen, und es werden wieder gewaschene und gegebenenfalls zerkleinerte Blätter der Bulbine frutescens, die aus Verfahrensschritt (1) erhalten wurden, in die Presse gefüllt. Bevorzugt wird die Presse wieder auf dieselbe Füllhöhe wie vor Durchführung der Pressstufe (I) aufgefüllt. In der Pressstufe (II) wird dann das frische Blattmaterial und das bereits gepresste Blattmaterial zusammen bei einem Druck im Bereich von etwa 50 bis 200 kPa, bevorzugter etwa 70 bis 180 kPa, noch bevorzugter 80 bis 150 kPa, insbesondere bei etwa 100 kPa unter Erhalt von weiterem Pflanzensaft (Pflanzensaft II) gepresst. Dann wird der Pressvorgang erneut unterbrochen. Schließlich werden in Pressstufe (III) wieder aus Verfahrensschritt (1) erhaltene frische Blätter zugegeben und die Pressvorrichtung entsprechend aufgefüllt. Der Pressdruck in Pressstufe (III) liegt nun bei einem Druck im Bereich von etwa 200 bis 400 kPa, bevorzugter etwa 250 bis 350 kPa, noch bevorzugter 270 bis 320 kPa, insbesondere bei etwa 300 kPa. Es wird für eine vorbestimmte Zeit unter Erhalt von Pflanzensaft (Pflanzensaft III) gepresst.

Der Pressdruck wird somit bevorzugt in den Stufen I, II und III sukzessive gesteigert. Die vorbestimmte Zeitdauer hängt von der Art und Größe der Presse, dem Befüllungsgrad und Art des Pflanzenmaterials ab und kann von einem Fachmann aus dem Stand der Technik ohne weiteres bestimmt werden. Als Faustregel kann eine Zeitdauer jeweils für jede Pressstufe im Bereich von einigen Minuten bis 2 Stunden, bevorzugter etwa 5 Minuten bis 1,5 Stunden, noch bevorzugter etwa 10 Minuten bis etwa 1 Stunde, ganz besonders bevorzugt etwa 10 Minuten bis 30 Minuten angegeben werden. Diese Zeitdauer kann im Einzelfall aber auch unter- oder überschritten werden.

Der Vorteil dieses dreistufigen Press-Verfahrens, bestehend aus einer Abfolge von 3 Pressschritten, unter Einhaltung vorgegebener Drücke und wiederholter Zugabe von frischem Blattmaterial, liegt darin, dass hierdurch eine signifikant höhere Ausbeute an Bulbine-frutescens-Pflanzensaft erreicht werden kann, als durch ein herkömmliches einstufiges Press-Verfahren.

Nach dem Pressen wird der Pflanzensaft der Bulbine frutescens erhalten, d.h. nach jedem Pressvorgang wird jeweils Pflanzensaft (Pflanzensaft 1 aus Press-Vorgang 1 und Pflanzensaft 2 aus Press-Vorgang 2, wobei Pflanzensaft 1 = Pflanzensaft I + Pflanzensaft II + Pflanzensaft III bei Verwendung der 3 Pressstufen) erhalten, der in Verfahrensschritt (5) zusammengegeben wird. Der in den Pressstufen gewonnene Pflanzensaft I, Pflanzensaft II und Pflanzensaft III kann auch bereits unmittelbar nach dem Durchführen der 3 Pressstufen zusammengesammelt oder zusammengegeben werden oder Pflanzensaft I, Pflanzensaft II und Pflanzensaft III können erst zu Verfahrensschritt (5) zusammengegeben werden. Der gewonnene Pflanzensaft ist nahezu farblos, mit einem leicht gelblichen Farbton und ist viskos oder leicht viskos, wobei die Viskosität u.a. vom Zeitpunkt der Ernte und vom Alter der Pflanze abhängt.

Bevorzugt wird der erhaltene Pflanzensaft nach dem Zusammengeben durch ein oder mehrere Filter filtriert, dies ist jedoch nicht in jedem Fall erforderlich und hängt von der Reinheit des eingesetzten Pflanzenmaterials ab. Besonders bevorzugt werden mehrere Filter nacheinander eingesetzt, ganz besonders bevorzugt ist eine Filterkaskade aus 2 bis 5 Filtern, bevorzugt 2 bis 4 Filtern, insbesondere 3 Filtern jeweils mit abnehmender Maschenweite vom ersten zum letzten Filter.

Bei einer bevorzugten Filterkaskade von 3 Filtern werden die Maschenweiten der Filter wie folgt gewählt: Bevorzugte Maschenweiten eines ersten Filters liegen im Bereich von 50 bis 200 µm, bevorzugt 70 bis 180 µm, insbesondere 100 bis 150 µm. Bevorzugte Maschenweiten eines zweiten Filters liegen im Bereich von 1 bis 100 µm, bevorzugt 5 bis 80 µm, insbesondere 10 bis 50µm. Bevorzugte Maschenweiten eines dritten Filters liegen im Bereich von 0,5 bis 5 µm, bevorzugt 1 bis 3,5 µm, insbesondere 1 bis 2,5 µm.

Im sich anschließenden Verfahrensschritt (6) wird der erhaltene Pflanzensaft pasteurisiert. Unter Pasteurisierung wird im Rahmen der Erfindung eine kurzzeitige Erwärmung zur Abtötung von Mikroorganismen im vorliegenden Pflanzensaft verstanden. Die Pasteurisierung erfolgt vorzugsweise in einem Temperaturbereich von etwa 50 bis 100°C, bevorzugter 60 bis 90°C, noch bevorzugter 70 bis 90°C. Es wird in der Regel für sehr kurze Zeit pasteurisiert; eine Zeitdauer von wenigen Sekunden bis Minuten, bevorzugter etwa 10 Sekunden bis 2 Minuten, noch bevorzugter weniger als 1 Minute, insbesondere weniger als 45 Sekunden ist für das Pasteurisieren häufig bereits ausreichend. Dies kann der Fachmann anhand seines Fachwissens ohne weiteres bestimmen.

Nach der Pasteurisierung kann es vorteilhaft sein, den pH-Wert des erhaltenen Pflanzensafts unter Verwendung einer pharmazeutisch oder kosmetisch akzeptablen Säure auf etwa 3,0 bis 6,0, bevorzugter etwa 3,5 bis 5,5, noch bevorzugter etwa 4,0 bis 5,5, insbesondere etwa 4,5 bis 5,0 einzustellen. Der Begriff "pharmazeutisch oder kosmetisch akzeptabel" bedeutet im Rahmen der vorliegenden Erfindung Verbindungen, welche für die beschriebene Behandlung eingesetzt werden können und aus medizinischer oder kosmetischer Sicht nicht unerwünscht sind, die im Rahmen einer medizinischen oder kosmetischen Verabreichung an einen Verwender bei Kontakt mit der Haut- oder Schleimhaut keinen nachteiligen und/oder schädlichen Einfluss zeigen, beispielsweise keine Reizungen, Allergien oder andere Probleme beim Verwender in Anbetracht der Schwere der Beeinträchtigung oder Erkrankung und der Notwendigkeit der Behandlung auslösen. Pharmazeutisch oder kosmetisch akzeptable Säuren sind beispielsweise ausgewählt aus organischen Säuren. Beispielhaft seien genannt: Adipinsäure, Alginsäure, Ascorbinsäure, Isoascorbinsäure (Erythorbinsäure), Asparaginsäure, Benzolsulfonsäure, Bernsteinsäure, 2-Acetoxybenzoesäure, Camphersäure, Camphersulfonsäure, Zimtsäure, Citronensäure, insbesondere Citronensäure-Monohydrat, Digluconsäure, Ethansulfonsäure, Glutaminsäure, Glykolsäure, Heptansäure, Hexansäure, Fumarsäure, 2-Hydroxyethansulfonsäure (Isethionsäure), Milchsäure, Maleinsäure, Hydroxymaleinsäure, Äpfelsäure, Malonsäure, Mandelsäure, Pamoasäure, Pectinsäure, Phenylessigsäure, 3-Phenylpropionsäure, Picrinsäure, Pivalinsäure, Pyruvinsäure, Stearinsäure, Sulfanilsäure, Weinsäure, p-Toluolsulfonsäure und Mischungen dieser. Besonders bevorzugt ist Citronensäure, insbesondere Citronensäure-Monohydrat.

Nach Zugabe der pharmazeutisch oder kosmetisch akzeptablen Säure ist es besonders zweckmäßig, den Pflanzensaft zu homogenisieren. Dies kann beispielsweise durch Rühren erfolgen, um alle Komponenten vollständig zu lösen, damit keine Aggregate mehr vorhanden sind. Das Rühren kann mit einer beliebigen Rührvorrichtung durchgeführt werden. Besonders bevorzugt ist ein Planetenrührer. Das Homogenisieren kann auch in anderer Weise erfolgen, beispielsweise durch Schütteln.

Die Kontrolle des mikrobiologischen Zustands und das Vorhandensein von pathogenen Organismen im erhaltenen Pflanzensaft der Erfindung erfolgt entsprechend den in verschiedenen Arzneibüchern beschriebenen Methoden, wie dem Europäischen Pharmakopöe und dem United States Pharmacopoeia. Die aerobe Keimzahl liegt bevorzugt bei weniger als 100 CFU/g, vorzugsweise bei maximal 10 CFU/g, und es sollten im Wesentlichen keine Pathogene vorhanden sein.

Es ist anzumerken, dass es zweckmäßig ist, den gewonnene Bulbine-frutescens-Pflanzensaft gegen opportunistische Mikroorganismen ("Mitläufer") zu schützen. Govender, du Plessis-Stoman, Downing und Van De Venter (South African Journal of Science, 102, 253 (2006)) kamen zu dem Schluss, dass Produkte, die auf lokalen Märkten angeboten werden, mikrobiologisch kontaminiert waren und als Gesundheitsrisiko für den Menschen zu betrachten sind.

Vorteilhafterweise werden daher dem erfindungsgemäßen Pflanzensaft ein oder mehrere pharmazeutisch oder kosmetisch akzeptable Konservierungsmittel zugesetzt. Der Begriff "pharmazeutisch oder kosmetisch akzeptabel" bedeutet im Rahmen der vorliegenden Erfindung Verbindungen, welche für die beschriebene Behandlung eingesetzt werden können und aus medizinischer oder kosmetischer Sicht nicht unerwünscht sind, die im Rahmen einer medizinischen oder kosmetischen Verabreichung an einen Verwender bei Kontakt mit der Haut- oder Schleimhaut keinen nachteiligen und/oder schädlichen Einfluß zeigen, beispielsweise keine Reizungen, Allergien oder andere Probleme beim Verwender in Anbetracht der Schwere der Beeinträchtigung oder Erkrankung und der Notwendigkeit der Behandlung auslösen. Pharmazeutisch oder kosmetisch akzeptable Konservierungsmittel sind beispielsweise: Benzoesäure, Sorbinsäure, Parabene (Methyl-, Ethyl-, Propyl-, Butyl-, Isobutyl- oder Benzyl-Paraben), Ameisensäure, Essigsäure, Propionsäure, Salicylsäure, p-Anissäure, Lävulinsäure und deren wasserlösliche Salze, Glycerol-/Polyglycerolmonoester von Caprylsäure, Caprinsäure-Laurinester, spezielle Diole, wie 1,2-Dihydroxypentan, 1,2-Dihydroxyhexan oder 1,2-Dihydroxyoctan, oder 1-(2-Ethylhexyl)-Glycerylether und dergleichen. Es können auch Mischungen verwendet werden.

Der erfindungsgemäß gewonnene Bulbine-frutescens-Pflanzensaft kann so verwendet werden, wie zuvor beschrieben hergestellt, oder dieser kann erst nach einer Weiterverarbeitung eingesetzt werden. Die Weiterverarbeitung kann ausgewählt sein aus ein oder mehreren der nachfolgenden Verfahrensweisen, die auch in beliebiger Reihenfolge kombiniert werden können: Filtrieren, Wasserverdampfung unter vermindertem Druck und/oder erhöhter Temperatur, azeotrope Destillation unter vermindertem Druck und/oder erhöhter Temperatur unter Verwendung eines geeigneten Co-Lösemittels, Sprühtrocknen oder Gefriertrocknen. Der Nachteil von Verfahren unter erhöhter Temperatur besteht in dem Risiko der Umwandlung der Anthrachinonglycoside in die entsprechenden Aglykone, die in Wasser schwer löslich oder unlöslich sind und so die Wirkung des Bulbine-frutescens-Pflanzensafts vermindern. Die Gefriertrocknung hat den Vorteil, dass die Umwandlung der Anthrachinonglycoside in die entsprechenden Aglykone stark gehemmt wird. Das Vorhandensein von Aglykonen kann beispielsweise durch Lösen des unlöslichen Teils in Aceton und/oder DMSO mit nachfolgender Zugabe von Kaliumhydroxid getestet werden. Es zeigt sich eine intensive Rot-oder Magenta- oder Violettfärbung.

Im Stand der Technik sind einige Verfahrensweisen beschrieben worden, welche aus Pflanzenteilen gewonnene Gele durch Zugabe von Wasserstoffperoxid, das im Anschluss daran wieder entfernt wird, stabilisieren. So beziehen sich beispielsweise die US 4 178 372 und die US 3 892 853 auf entsprechend stabilisiertes Aole vera-Gel. Hierzu werden beispielsweise in der US 4 178 372 die Blätter der Aloe vera durch einen Filter gedrückt, auf eine Temperatur im Bereich von 35 bis 80°C erhitzt, H₂O₂ zugegeben, dann Ascorbinsäure zugesetzt, um das H₂O₂ zu zerstören und die katalytische Oxidation zu stoppen und durch Zugabe von Citronensäure den pH-Wert auf etwa 4 bis 6 einzustellen.

Derartige Verfahren haben jedoch einige Nachteile: Beispielsweise muss die zugegebene Wasserstoffperoxidmenge genau dosiert und kontrolliert werden, um keine Zerstörung des Produkts hervorzurufen. Aus diesem Grund werden beispielsweise manchmal nur katalytische Mengen an H₂O₂ verwendet. Weiterhin sind stets zusätzliche Schritte zur Entfernung von H₂O₂ aus dem stabilisierten Gel erforderlich, da ansonsten der Einsatz des Gels in Kosmetika oder Pharmaka nur eingeschränkt möglich ist. Auch muss zur Entfernung des Wasserstoffperoxids entsprechend schonend vorgegangen werden, um das stabilisierte Produkt nicht wieder zu zerstören. Außerdem bleiben möglicherweise nach wie vor Spuren an Wasserstoffperoxid im erhaltenen Produkt zurück, die nicht ohne weiteres entfernt werden können, so dass der Anwendungsbereich des Gels hierdurch eingeschränkt wird.

Erfindungsgemäß wird nun die Verwendung von Wasserstoffperoxid gänzlich vermieden, wobei in überraschender Weise dennoch ein stabiler und äußerst wirksamer Bulbine-frutescens-Pflanzensaft erhalten werden kann. Gemäß der vorliegenden Erfindung wird die Wasserstoffperoxid-Behandlung durch ein spezielles Pressverfahren, das aus einer Abfolge von verschiedenen Pressschritten aufgebaut ist, und einem anschließenden Pasteurisieren ersetzt. Das Wasserstoffperoxid wird im Stand der Technik in erster Linie dazu eingesetzt, um in dem aus den Blättern gewonnenen Gel die vorhandene Gelstruktur aufzubrechen. Die Gelstruktur ist auf die hochmolekularen und wohl auch verzweigten Polysaccharide zurückzuführen. Die Gelstruktur wirkt sich vor allem negativ auf die Verarbeitbarkeit in der Endproduktformulierung aus, z.B. bei der Formulierung in Kosmetika. Im Gegensatz zum gesamten bekannten Stand der Technik gelingt es nun erfindungsgemäß trotz des Weglassens von Wasserstoffperoxid und aufgrund der Durchführung eines speziellen Pressverfahrens, dass diese nachteiligen Verbindungen im gewonnen Pflanzensaft nicht mehr vorliegen. Da H₂O₂ im Allgemeinen zur Zerstörung dieser nachteiligen Spezies eingesetzt wird, ist dies im Rahmen der vorliegenden Erfindung nun nicht mehr erforderlich. Weiterhin werden die nachteiligen Enzymreste, die im Pflanzensaft vorhanden sind, erfindungsgemäß durch die nachgeschaltete Pasteurisierung deaktiviert. Diese Enzymreste sind problematisch, da diese zu einem Verlust der Wirkung beim erhaltenen Endprodukt, zu verstärkter Farbbildung und zur Glycolyse der Knipholonglycoside und deren Derivaten führen.

Somit wird potentiellen und nicht erwünschten Abbauprozessen vorgebeugt, obwohl kein H₂O₂ eingesetzt wird. Ein mikrobiologischer und/oder enzymatischer Abbau des Produkts wird durch ein nachgeschaltetes Pasteurisierungsverfahren vermieden. Ein entsprechend stabiler Pflanzensaft wird erhalten. Schließlich ermöglicht der Verzicht auf H₂O₂ einen uneingeschränkten Einsatz des erhaltenen Pflanzensafts in der Naturkosmetik, da auch Spurenverunreinigungen von H₂O₂ vermieden werden.

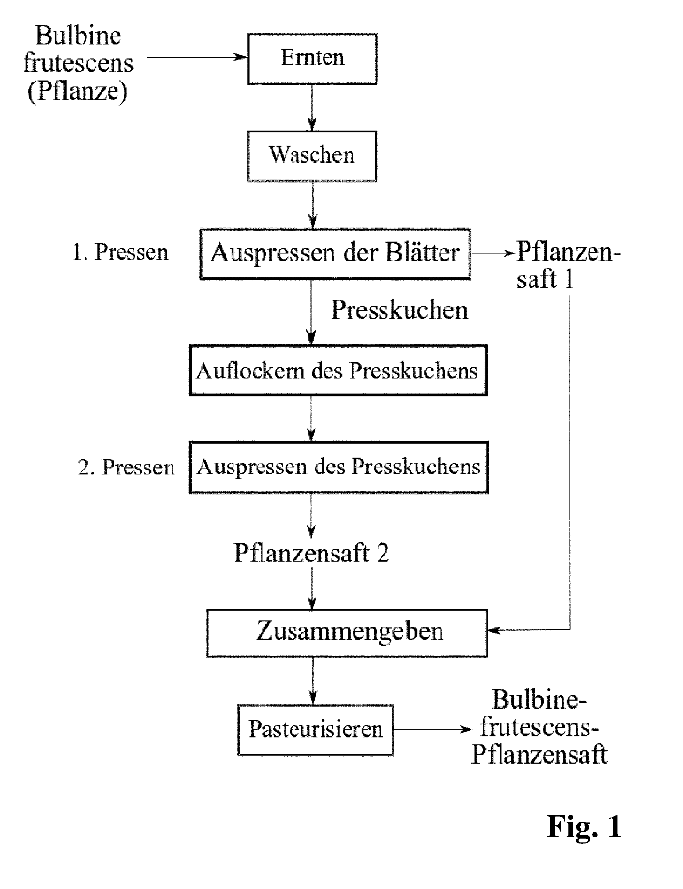

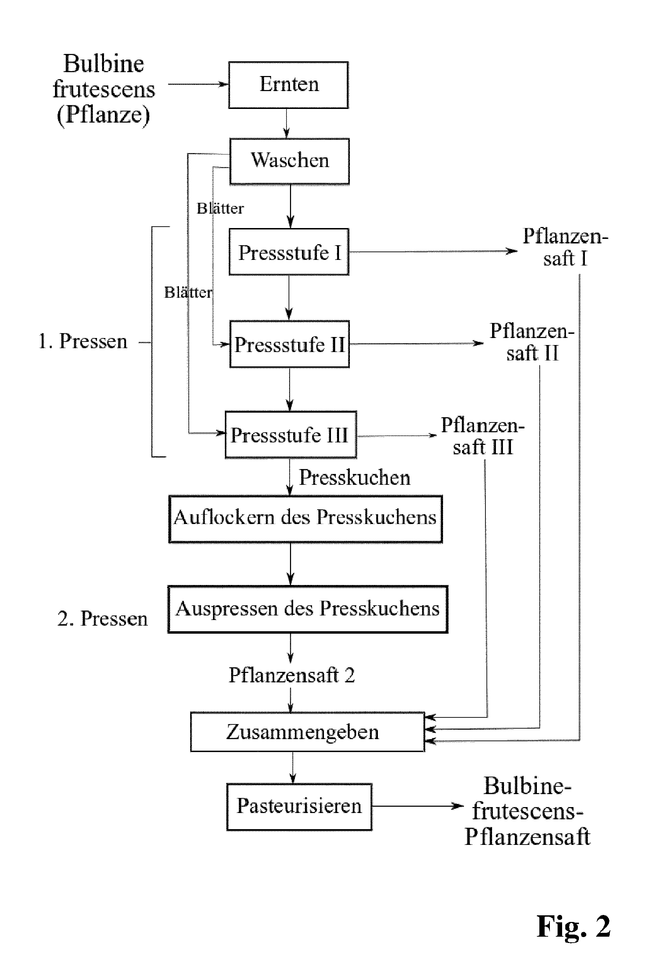

Das erfindungsgemäße Verfahren zur Herstellung des Bulbine-frutescens-Pflanzensafts der Erfindung wird nachfolgend anhand der Figuren 1 und 2 schematisch zusammengefasst, welche die vorliegende Erfindung nicht beschränken sollen. Es zeigt:
- Figur 1: ein schematisches, vereinfachtes Ablaufdiagramm einer Ausführungsform der vorliegenden Erfindung und
- Figur 2: ein weiteres schematisches, vereinfachtes Ablaufdiagramm einer weiteren Ausführungsform der vorliegenden Erfindung.

Figur 1 zeigt in einem Ablaufdiagramm die einzelnen Verfahrensschritte des erfindungsgemäßen Verfahrens gemäß einer bevorzugten Ausführungsform, wobei nach dem Ernten der Bulbine frutescens, die gewonnenen Pfanzenteile, insbesondere die Blätter, gewaschen werden. Hiernach erfolgt ein Auspressen der Blätter unter Erhalt von Pflanzensaft und einem Presskuchen (1. Pressen), wie bereits im Einzelnen beschrieben. Nach dem Aufschütteln und/oder Auflockern des Presskuchens, das bevorzugt außerhalb der Pressvorrichtung stattfindet, wird der erhaltene aufgelockerte Presskuchen ausgepresst (2. Pressen), das wie oben beschrieben durchgeführt wird. Dann wird der erhaltene Pflanzensaft aus dem 1. und dem 2. Pressen zusammengegeben und anschließend pasteurisiert.

Figur 2 ist eine weitere Variante des erfindungsgemäßen Verfahrens anhand eines Ablaufdiagramms in schematischer und vereinfachter Art und Weise dargestellt. Nach dem Ernten der Bulbine frutescens werden die gewonnenen Pflanzenteile, insbesondere die Blätter, gewaschen. Hiernach erfolgt ein Auspressen der Blätter (1. Pressen), das sich in 3 Pressstufen aufteilt. In der Pressstufe I wird bei einem Druck im Bereich von etwa 10 bis 100 kPa, bevorzugt etwa 20 bis 80 kPa, bevorzugter etwa 30 bis 70 kPa, insbesondere bei etwa 50 kPa gepresst. Hieraus wird der Pflanzensaft I erhalten. Danach wird der Pressvorgung unterbrochen und es werden aus Verfahrensschritt (1) gewaschene und gegebenenfalls zerkleinerte Blätter der Bulbine frutescens in die Pressvorrichtung gegeben. Beispielsweise ist es zweckmäßig die Pressvorrichtung wieder vollständig mit Blättern aufzufüllen. Dann wird in der Pressstufe II bei einem Druck im Bereich von etwa 50 bis 200 kPa, bevorzugter etwa 70 bis 180 kPa, noch bevorzugter 80 bis 150 kPa, insbesondere bei einem Druck von etwa 100 kPa gepresst und der Pflanzensaft II wird erhalten. Anschließend wird der Pressvorgang wieder unterbrochen und es werden wieder aus Verfahrensschritt (1) gewaschene und gegebenenfalls zerkleinerte Blätter der Bulbine frutescens in die Pressvorrichtung gegeben. Beispielsweise ist es zweckmäßig die Pressvorrichtung wieder vollständig mit Blättern aufzufüllen. Schließlich wird in der Pressstufe III erneut gepresst, aber diesmal bei einem Druck im Bereich von etwa 200 bis 400 kPa, bevorzugter etwa 250 bis 350 kPa, noch bevorzugter 270 bis 320 kPa, insbesondere bei etwa 300 kPa unter Erhalt des Pflanzensafts III. Der in Pressstufe III erhaltene Presskuchen wird dann wie bei Figur 1 erläutert aufgeschüttelt und/oder aufgelockert, bevorzugt außerhalb der Pressvorrichtung, und das weitere Auspressen des Presskuchens (2. Pressen) wird wie oben beschrieben durchgeführt und der Pflanzensaft 2 wird erhalten. Es ist zweckmäßig für alle Pressvorgänge dieselbe Pressvorrichtung einzusetzen, es können aber auch unterschiedliche Pressvorrichtungen zum Einsatz kommen.

Sämtliche erhaltenen Pflanzensäfte aus den einzelnen Press-Vorgängen werden dann zusammengegeben (d.h. Pflanzensaft 1 aus dem 1. Press-Vorgang und Pflanzensaft 2 aus dem 2. Press-Vorgang mit Pflanzensaft 1 = Pflanzensaft I + Pflanzensaft II + Pflanzensaft III), oder können teilweise bereits zuvor zusammengegeben worden sein. Sämtlicher erhaltener Pflanzensaft wird dann pasteurisiert.

Gegenstand der vorliegenden Erfindung ist auch die Verwendung des erfindungsgemäßen Bulbine-frutescens-Pflanzensafts im therapeutischen oder kosmetischen Bereich.

Die Verwendung im kosmetischen Bereich umfasst die Körper- und Schönheitspflege, d.h. die Erhaltung, Wiederherstellung oder Verbesserung der Schönheit des menschlichen Körpers, beispielsweise durch Reinigen, Stabilisieren, Vitalisieren und/oder Deodorieren der Haut und Reinigen, Stabilisieren und/oder Vitalisieren der Schleimhaut.

Die Verwendung im therapeutischen Bereich umfasst insbesondere die Anwendung des Bulbine-frutescens-Pflanzensafts zur Behandlung oder Vorbeugung von Krankheiten und Verletzungen der Haut und Schleimhaut. Auch Erkrankungen der Hautanhangsgebilde, wie Haare, Nägel, Talg- und Schweißdrüsen, werden zu den Hautkrankheiten gezählt, da diese auch von epidermaler Abstammung sind.

Bekanntermaßen verweisen die bisherigen Veröffentlichungen in der Literatur darauf, dass im Wesentlichen keine antimikrobiellen Eigenschaften der Bulbine frutescens vorhanden sein sollen. In überraschender Weise wurde nun in der vorliegenden Erfindung festgestellt, dass der erfindungsgemäße Bulbine-frutescens-Pflanzensaft über unerwartete antimikrobielle Eigenschaften verfügt. Die Versuche, die nachfolgend im experimentellen Teil beschrieben sind, zeigten Ergebnisse, die äußerst überraschend, völlig unerwartet und im Widerspruch zu früheren, in der Literatur angegebenen Daten sind. Die nachgewiesenen antimikrobiellen Eigenschaften des erfindungsgemäßen Bulbine-frutescens-Pflanzensafts ermöglichen die Bereitstellung von Produkten mit Vorteilen im Hinblick auf besondere antimikrobielle Anforderungen. Die Versuche zeigten auch, dass der Bulbine-frutescens-Pflanzensaft zwar kein generell wirksames antimikrobielles Mittel darstellt, da die völlige Abtötung bestimmter Organismen nicht stets erreicht wird, aber es stellt einen funktionellen Inhaltsstoff dar, der eine unerwartete antimikrobielle Wirkung in bestimmtem Maße bereitstellt.

Weiter Versuche haben gezeigt, dass sich Bulbine-frutescens-Pflanzensaft, angereichert mit einer kleinen Menge Wasserstoffperoxid (z.B. 0,05 Gew.-%, ausgedrückt als H₂O₂), gegen bestimmte Organismen wie ein Desinfektionsmittel verhält, insbesondere gegen Staphylococcus aureus, einschließlich Methicillinresistenten Staphylococcus aureus (MRSA) und Clindamycin-, Erythromycin- und Ciprofloxacin-resistenten Staphylococcus aureus (CEC-SA). Diese resistenten Stränge sind für gewöhnlich schwer zu kontrollieren, insbesondere in einer Krankenhausumgebung. Die Tests haben außerdem gezeigt, dass viele andere Organismen durch die beschriebene Zusammensetzung nicht oder nur eingeschränkt beeinflusst werden, insbesondere die natürliche Mikroflora der Haut und der Schleimhäute, wodurch die natürliche Mikroflora der Haut und der Schleimhäute geschont werden kann. Daher eignet sich Bulbine-frutescens-Pflanzensaft, insbesondere angereichert mit einer kleinen Menge Wasserstoffperoxid (z.B. 0,05 Gew.-%, ausgedrückt als H₂O₂), insbesondere zur Desinfektion von Operationssälen.

Vergleichsversuche, bei denen Wasserstoffperoxid (z.B. 0,05 Gew.-%, ausgedrückt als H₂O₂) allein getestet wurde, erbrachten im Gegensatz zu den Versuchen von Bulbine-frutescens-Pflanzensaft, angereichert mit Wasserstoffperoxid (z.B. 0,05 Gew.-%, ausgedrückt als H₂O₂), nicht die abtötenden Eigenschaften. Es wird daher angenommen, dass die Zugabe von Wasserstoffperoxid in kleinen Mengen im Bereich von >0 Gew.-% bis < 0,1 Gew.-% H₂O₂, zum Bulbine-frutescens-Pflanzensaft eine synergistische Wirkung hat, da diese die Summe der beiden Einzelwirkungen deutlich übersteigt.

Der Bulbine-frutescens-Pflanzensaft besteht aus vielen verschiedenen chemischen Einheiten, und dazu gehören auch Polysaccharide. Diese haben sich als nützlich bei der Wundheilung erwiesen, wie von Pather (Thesis, 2009) demonstriert. Es wurden daher *In-vitro*-Studien durchgeführt, um die Zytotoxizitäts- und Zellproliferationswirkungen des Bulbine-frutescens-Pflanzensafts zu bestimmen. Der Pflanzensaft weist keine zytotoxischen Eigenschaften auf und die Zellteilung war besser als 100 % bei 0,1 µg/ml. Weiterhin wurde festgestellt, dass der Bulbine-frutescens-Pflanzensaft eine besonders starke wundheilende Wirkung bei Verletzungen der Haut- und Schleimhaut aufweist. Biochemische Analysen von behandeltem Wundgewebe zeigten im Vergleich zu unbehandelten Wunden eine deutlich erhöhte Menge Collagen, Einweiß und DNA-Gesamtgehalt. Ausgeschnittene und eingeschnittene Wunden in Schweinehaut zeigten im Vergleich zu unbehandelten Wunden eine stark erhöhte Geschwindigkeit beim Wundverschluss und eine vollständige Reepithelialisierung. Eine gute Wundheilung ist wichtig, da klinische Infektionen auftreten können, wenn die Bakterienkolonien in und um die Wundstellen auf ein kritisches Maß anwachsen. Dies kann zu einer Verschlechterung des Wundzustandes und, wenn systemische Infektionen auftreten, für den Patienten zu grippeähnlichen Symptomen mit Fieber führen. Häufig sind systemische Antibiotika erforderlich und wesentlicher Bestandteil der Behandlung. Bei frühzeitiger Behandlung mit dem Bulbine-frutescens-Pflanzensaft kann aufgrund eines günstigen Verlaufs bei der Wundheilung daher auf die Verabreichung von Antibiotika verzichtet werden.

Der Bulbine-frutescens-Pflanzensaft kann auch vorbeugend zur Vermeidung von Wunden, beispielsweise Brandwunden, oder zur schnellen Heilung von (Brand-)Wunden eingesetzt werden. Der Bulbine-frutescens-Pflanzensaft ist außerdem besonders erfolgreich bei der Wundheilung kleiner Wunden anwendbar, die beispielsweise bei der Rasur und der Entfernung von Haaren und von einwachsenden Haaren bei Männern mit stark gekraustem Haar entstehen, bei schlecht heilenden Wunden, die beispielsweise durch eine Infektion durch Methicillinresistenten Staphylococcus aureus (MRSA) und Clindamycin-, Erythromycin- und Ciprofloxacin-resistenten Staphylococcus aureus (CEC-SA) (besonders an den Füßen) entstanden sind, und auch bei anderen kleinen Wunden.

Der Bulbine-frutescens-Pflanzensaft hat sich außerdem bei der Behandlung von Furunkeln und Karbunkeln bewährt.

Weiterhin wird angenommen, dass Arabinogalactane (AGs) die wichtigsten Polysaccharide sind. Diese Polysaccharide sind aus Arabinose und Galactose (Monosaccharide) zusammengesetzt; Arabinogalactane werden von Pflanzen erzeugt. Arabinogalactane sind zum Beispiel in großen Mengen in Gummi arabicum und Gummi ghatti zu finden. Außerdem erzeugen bestimmte Mikroorganismen Arabinogalactane. An Arabinogalactan angehängte Proteine (AGP) dienen als Signalmoleküle zwischen den Zellen sowie als Kleber zum Verschließen von Wunden an Pflanzen. Arabinogalactane sind dafür bekannt, das Immunsystem von Pflanzen zu stimulieren, da sie die Aktivität natürlicher Killerzellen und anderer Komponenten des Immunsystems steigern (A. M. Showalter, Cell. Mol. Life Sci., 58, 1399 (2001)). Obwohl dies besonders für Pflanzen gilt, können sie eventuell auch den menschlichen Körper beim Kampf gegen Infektionen unterstützen, wie von Yamada & Kiyohara ("Complement-activating polysaccharides from medicinal herbs", Immuno-modulatory Agents from Plants, 161 - 202 (1999), H. Wagner (Hg.), Birkhäuser Basel) vorgeschlagen.

Durch Versuche konnte nun gezeigt werden, dass der Bulbine-frutescens-Pflanzensaft tatsächlich hochgradig immunstärkende Eigenschaften aufweist. Dies belegen Versuche, bei denen der Bulbine-frutescens-Pflanzensaft bei der Wundheilung immungeschwächter Personen getestet wurde, insbesondere bei Patienten mit Diabetes Typ II mit schlechter Wundheilung an den unteren Extremitäten und Füßen. Auch bei Wunden, die bereits seit längerer Zeit bestanden, konnte mit dem erfindungsgemäßen Pflanzensaft eine Wundheilung erreicht werden, wenn auch zum Teil unter Ausbildung einer Narbe. Der Bulbine-frutescens-Pflanzensaft kann daher auch bei der Wundheilung schlecht heilender Wunden, wie etwa bei Patienten mit Diabetes Typ II oder AIDS-Patienten, erfolgreich eingesetzt werden.

Gegenstand der vorliegenden Erfindung ist daher auch die Verwendung des Bulbine-frutescens-Pflanzensafts als Mittel zur Stärkung der Immunabwehr.

Durch die Kombinationswirkung des Bulbine-frutescens-Pflanzensafts, das neben der heilenden Wirkung auch über immunstärkende Eigenschaften verfügt, wird der Bulbine-frutescens-Pflanzensaft daher zur Vermeidung/Vorbeugung und schnellen Heilung besonders erfolgreich eingesetzt.

Der erfindungsgemäße Bulbine-fructescens-Pflanzensaft kann auch wirksam zur Bekämpfung von Nagel- und Fußpilz eingesetzt werden. Nagel- und Fußpilz werden durch Hefepilze verursacht, Infektionen durch anthropophile Erreger, wie beispielsweise Trichophyton rubrum, verlaufen in der Regel weniger heftig, dafür aber chronischer als Infektionen mit zoophilen Erregern, wie Trichophyton mentagrophytes. Diese Organismen sind durch direkten oder indirekten Kontakt mit infizierten Personen leicht übertragbar. Die Organismen sind gewöhnlich schwer zu eliminieren, da sie nicht nur die Oberfläche der Haut oder des Nagels besiedeln, sondern auch im subkutanen Gewebe vorhanden sind. Die meisten Fungizide, wie Clotrimazol, Terbinafin und Miconazol, weisen nur eine stark beschränkte Penetrationstiefe auf, was zu einem hohen Maß an Rückfällen führt. Die Wirksamkeit dieser Mittel ist deshalb recht eingeschränkt.

Der Bulbine-frutescens-Pflanzensaft verstärkt jedoch in unerwarteter Weise den transdermalen Transport (auch bei Nägeln), wie durch Tests mit der Franz-Diffusionszelle nachgewiesen wurde. Außerdem kann der transdermale Transport durch die Verwendung eines Penetrationsverstärkers, wie etwa Polysorbat 80, ungesättigtes Phosphatidylcholin und ähnlichen Phospholipiden, Phytantriol und anderen Produkten, weiter verbessert werden, wie beispielsweise von Benson (Current Drug Delivery, 2, 23 (2005)) beschrieben.

Der Bulbine-frutescens-Pflanzensaft konnte auch bei Kandidose erfolgreich eingesetzt werden. Der Hefepilz Candida albicans und verwandte Arten sind für verschiedene Arten von Kandidose verantwortlich, wie beispielsweise topische oder vaginale Kandidose. Die Behandlung mit herkömmlichen pharmazeutischen Produkten führt zu einer hohen Rückfallrate, insbesondere bei immungeschwächten Personen. Der Candida albicans kann mit Bulbine-frutescens-Pflanzensaft wirksam behandelt werden.

Gegenstand der vorliegenden Erfindung ist daher auch der Bulbine-frutescens-Pflanzensaft zur Verwendung bei der Behandlung von Haut- oder Schleimhauterkrankungen, insbesondere Infektionen oder Entzündungen der Haut oder Schleimhaut, wie Pilzerkrankungen, oder bei der Behandlung oder Vorbeugung von Verletzungen der Haut oder Schleimhaut, wie (Brand-)Wunden.

Der Ausdruck "Bulbine-frutescens-Pflanzensaft" oder "Pflanzensaft" wie in der vorliegenden Erfindung verwendet, bedeutet nicht zwangsläufig, dass die Darreichungsform oder Formulierung per se einen Saft darstellt. Vielmehr soll dies im Rahmen der Erfindung so verstanden werden, dass eine beliebige Darreichungsform oder Formulierung gewählt werden kann, die den "Pflanzensaft" oder ein hieraus entsprechend weiterverarbeitetes Produkt als Wirkbestandteil enthält.

Weiterhin hat sich gezeigt, dass der Bulbine-frutescens-Pflanzensaft auch in vorteilhafter Weise für die Körperpflege, beispielsweise in Deodorants, als hoch wirksamer Bestandteil eingesetzt werden kann. Direkt nach dem Ausscheiden ist der menschliche Schweiß geruchlos. Im Schweiß vorhandene Sterolester unterliegen jedoch einer mikrobiologischen Hydrolyse, wobei neben kurzkettigen Carbonsäuren (Essigsäure, Baldriansäure, Buttersäure und Isobuttersäure) Androstene gebildet werden. Androstene und kurzkettige Carbonsäuren sind verantwortlich für den wahrnehmbaren unerwünschten Geruch. Die Organismen, die für die Hydrolyse der Androstenester verantwortlich sind, werden oft der Familie der Lactobacillaceae, Milchsäure erzeugenden Organismen, zugeordnet, können aber auch andere gram-positive oder gram-negative Bakterien sein, wie etwa Staphylococcus epidermides. Durch Verringerung oder Beseitigung dieser Organismen kann der unangenehme Geruch vermindert oder vollständig beseitigt werden. Übliche deodorisierend wirkende Formulierungen, die zusätzlich den erfindungsgemäßen Bulbine-frutescens-Pflanzensaft enthalten, zeigen eine besonders hohe Wirksamkeit. Die Wirksamkeit derartiger Formulierungen wird durch den Aufbau der Hautbarriere weiter verbessert.

Ein weiterer Anwendungsbereich des erfindungsgemäßen Bulbine-fructescens-Pflanzensafts ist bei der Mundpflege. In jüngerer Zeit hat sich gezeigt, dass eine gestörte orale Mikroflora Herz-Kreislauferkrankungen und Fehlgeburten verursachen kann. Der Bulbine-frutescens-Pflanzensaft leistet bei der Regulierung der oralen Mikroflora einen äußerst wirksamen Beitrag, wobei der Mundgeruch als Gradmesser dienen kann. In einigen Fällen tragen Schwefelwasserstoff, Methylmercaptan und Dimethylsulfid neben verschiedenen alkylierten Aminen wesentlich zum Mundgeruch bei. In Versuchen wurde gezeigt, dass durch Ausspülen der Mundhöhle mit einer 10%igen wässerigen Lösung des Bulbine-frutescens-Pflanzensafts unerwünschter Mundgeruch vollständig eliminiert werden konnte. Kleine Infektionen und Entzündungen des Zahnfleisches und der Mundschleimhaut verschwinden. Der Bulbine-frutescens-Pflanzensaft ist damit herkömmlichen pharmazeutischen Präparaten, einschließlich der auf Chlorhexidindiglukonat und Wasserstoffperoxid basierenden Präparate zur Munddesinfektion, überlegen. S. salivarius wird vom Bulbine-frutescens-Pflanzensaft kaum beeinträchtigt; demgegenüber sind Chlorhexidin und Wasserstoffperoxid für die kommensale Mikroflora in der Mundhöhle stark schädigend.

Es wurde bereits beschrieben, dass der Bulbine-frutescens-Pflanzensaft das Immunsystem stärkt, was wahrscheinlich hauptsächlich auf die vorhandenen Polysaccharide zurückzuführen ist. Eine weitere Anwendung, die wahrscheinlich hiermit in Zusammenhang steht, ist die als Anti-Ageing-Produkt. Hierbei kommt es darauf an, dass ein positiver Einfluss auf beispielsweise die Hautelastizität, die Hautfestigkeit, den Wasserhaushalt der Haut sowie die Zelldifferenzierung und -proliferation vorliegt. Die Versuche zeigten nun, dass der Bulbine-frutescens-Pflanzensaft, beispielsweise formuliert als Creme, zu einer deutlich verbesserten Elastizität und einer signifikant verbesserten Festigkeit der Haut führte, wobei gleichzeitig der Wasserverlust des subkutanen Hautgewebes stark verringert wurde. Es wurde auch ein deutlich verbessertes Hautbild beobachtet.

Die vorliegende Erfindung betrifft auch eine kosmetische oder pharmazeutische Zusammensetzung, umfassend den Bulbine-frutescens-Pflanzensaft gemäß der vorliegenden Erfindung, in Mischung mit einem oder mehreren pharmazeutisch oder kosmetisch akzeptablen Trägermedien oder Hilfsstoffen. Pharmazeutisch oder kosmetisch akzeptable Träger und Hilfsstoffe sind dem Fachmann im Stand der Technik bekannt. Diese Träger und Hilfsstoffe umfassen beispielsweise lonen-Austauscher, Emulgatoren, Bindemittel, Suspendierhilfsmittel, Stabilisatoren, bakteriostatische Mittel, Antioxidantien, Lecithin, Serumproteine, Puffersubstanzen, pH-Einstellmittel, Konservierungsmittel, Wasser, Salze oder Elektrolyte, Substanzen auf Cellulosebasis und zahlreiche andere kosmetisch oder pharmazeutisch akzeptable Additive.

Zur therapeutischen oder kosmetischen Anwendung kann der Pflanzensaft in irgendeiner herkömmlichen Dosierungsform in irgendeiner herkömmlichen Art und Weise auf der Haut oder Schleimhaut verabreicht werden. Bevorzugte Verabreichungswege sind transdermal und topisch. Die Dosierung des Bulbine-frutescens-Pflanzensafts hängt u.a. ab von der Verabreichungsform, dem Verwendungszweck, der zu behandelnden Beeinträchtigung, Störung oder Erkrankung, dem Verwender, und den speziellen ausgewählten Trägern und/oder Hilfsstoffen. Erfindungsgemäß wird der Pflanzensaft in der gewählten Darreichungsform bzw. Formulierung insbesondere durch äußere, lokale Auftragung verabreicht. Die Darreichungsform bzw. Formulierungen sind daher für eine transdermale oder topische Verabreichung ausgewählt und angepasst. Derartige Formulierungen werden in einer flüssigen, halbfesten oder festen Dosierungsform bereitgestellt und können beispielsweise in Form einer Lösung, beispielsweise als Tropfen, Tinktur, Öl oder Spüllösung, Suspension, Emulsion, Creme, Lotion/Milch, Schüttelmixtur, Puder, Paste, Spray, in transdermalen Pflastern oder dergleichen vorliegen.

Die Vorteile der vorliegenden Erfindung sind außerordentlich vielschichtig:
So wird der erfindungsgemäße Bulbine-frutescens-Pflanzensaft durch ein speziell entwickeltes Verfahren aus den Blättern der Bulbine frutescens gewonnen, einem speziellen Pressverfahren unterzogen und anschließend pasteurisiert. Der erhaltene Pflanzensaft kann so wie es ist formuliert werden oder es wird einer entsprechenden Weiterverarbeitung unterzogen und dann in eine geeignete Formulierungsform einbezogen.

Der erfindungsgemäße Pflanzensaft kann sowohl im kosmetischen als auch therapeutischen Bereich Verwendung finden. Die Verabreichungsform ist hierbei nicht besonders beschränkt, wobei der Pflanzensaft in der gewählten Formulierung zweckmäßiger Weise äußerlich auf der zu behandelnden Stelle örtlich aufgetragen wird. Der bevorzugte Verabreichungsweg ist transdermal oder topisch, wobei die den Pflanzensaft enthaltende Formulierung bevorzugt lokal auf die Haut- oder Schleimhaut aufgetragen wird.

In völlig unerwarteter Weise konnte festgestellt werden, dass der erfindungsgemäße Bulbine-frutescens-Pflanzensaft über antimikrobielle Eigenschaften verfügt, so dass entsprechende Produkte bereitgestellt werden können. Der Bulbine-frutescens-Pflanzensaft kann, angereichert mit einer kleinen Menge Wasserstoffperoxid (z.B. 0,05 Gew.-%, ausgedrückt als H₂O₂) sogar als Desinfektionsmittel eingesetzt werden. Ein derartiges Kombinations-Desinfektionsmittel wirkt sogar gegen Staphylococcus aureus, einschließlich Methicillin-resistentem Staphylococcus aureus (MRSA) und Clindamycin-, Erythromycin- und Ciprofloxacin-resistentem Staphylococcus aureus (CEC-SA).

Der erfindungsgemäße Bulbine-frutescens-Pflanzensaft kann in vorteilhafter Weise zur therapeutischen Behandlung von Erkrankungen der Haut und Schleimhaut eingesetzt werden, insbesondere gegen Infektionen und Entzündungen der Haut und Schleimhaut, wie Pilzerkrankungen, beispielsweise Fuß- und Nagelpilz oder Kandidose, insbesondere topische oder vaginale Kandidose, Verletzungen der Haut und Schleimhaut, wie Wunden, insbesondere Brandwunden, und kann beispielsweise auch zur Behandlung von Furunkeln und Karbunkeln eingesetzt werden. Der erfindungsgemäße Pflanzensaft ist auch vorbeugend wirksam, beispielsweise um Verletzungen und/oder Wunden der Haut oder Schleimhaut zu verhindern.

Der Pflanzensaft kann auch zur Stärkung der Immunabwehr verabreicht werden. Dies spielt insbesondere bei Patienten mit einem geschwächten Immunsystem eine Rolle, wie beispielsweise bei Diabetes- oder AIDS-Patienten. Durch die Kombinationswirkung des Bulbine-frutescens-Pflanzensafts, der neben einer heilenden Wirkung auch über immunstärkende Eigenschaften verfügt, wird der Bulbine-frutescens-Pflanzensaft zur Vermeidung/Vorbeugung und schnellen Heilung besonders erfolgreich eingesetzt.

Infektionen und Entzündungen klingen innerhalb kurzer Zeit ab, Wunden verheilen deutlich schneller und eine Immunstärkung findet statt, so dass Rückfälle weitgehend ausgeschlossen werden können.

Die gute Wundheilung durch den Bulbine-frutescens-Pflanzensaft kann dazu führen, dass auf die zusätzliche Verabreichung von Antibiotika verzichtet werden kann.

Im kosmetischen Bereich wird der erfindungsgemäße Pflanzensaft insbesondere zur Körperpflege, Mundpflege, in Deodorants und als Anti-Ageing-Produkt verwendet.

Die Wirkung ist zum Teil handelsüblichen Produkten weit überlegen, wobei die natürliche Mikroflora der Haut und der Schleimhäute nicht geschädigt wird, sondern vielmehr intakt erhalten bleibt.

Insbesondere in den Anwendungen im kosmetischen Bereich, beispielsweise als Anti-Ageing-Produkt, zeigt sich der positive Einfluß des erfindungsgemäßen Pflanzensafts auf die Haut und Schleimhaut. So steigt die Hautelastizität, die Hautfestigkeit nimmt zu, der Wasserhaushalt der Haut sowie die Zelldifferenzierung und -proliferation werden nachhaltig positiv beeinflusst. Außerdem wird das Erscheinungsbild der Haut deutlich verbessert.

Im nachfolgenden experimentellen Teil wird zunächst in einem Ausführungsbeispiel ein Verfahren zur Herstellung des erfindungsgemäßen Bulbine-frutescens-Pflanzensafts beschrieben. Im Anschluss daran sind beispielhaft einige Versuche detailliert geschildert, in denen die therapeutische und kosmetische Wirksamkeit des erfindungsgemäßen Bulbine-frutescens-Pflanzensafts gezeigt sind. Selbstverständlich soll die vorliegende Erfindung hierdurch nicht beschränkt werden.

### Ausführungsbeispiel

### Herstellung des erfindungsgemäßen Bulbine-frutescens-Pflanzensafts

### Ernten:

Die in den Morgenstunden frisch geernteten Blätter der Bulbine frutescens wurden zu einer Anlage transportiert, wo die Weiterverarbeitung erfolgte.

### Qualitätskontrolle:

Die Abstammung, der Zustand sowie die Sauberkeit des geernteten Pflanzenmaterials wurden überprüft und gegebenenfalls wurden Verunreinigungen aussortiert und entfernt.

### Waschen:

Die Blätter wurden mit entkeimtem Wasser gewaschen und anschließend zum Abtropfen auf einem Gestell ausgebreitet.

### Pressen:

Die nach dem Waschen erhaltenen Blätter wurden in eine hydraulische Korbpresse geladen und gepresst, um den Bulbine-frutescens-Pflanzensaft zu erhalten. Hierfür wurde in einer Pressstufe I die Korbpresse mit Pflanzenmaterial, insbesondere den Blättern, gefüllt und bei 50 kPa für etwa 25 min gepresst. Die Korbpresse wurde danach mit frischem Pflanzenmaterial, insbesondere den Blättern, aufgefüllt und erneut gepresst; diesmal bei 100 kPa für etwa 10 min (Pressstufe II). Im Anschluss daran wurde wieder frisches Pflanzenmaterial in die Korbpresse gefüllt und erneut gepresst. Der Pressdruck betrug in der Pressstufe III etwa 300 kPa und die Pressdauer war etwa 20 min.

Nach Beendigung dieser 3 Pressstufen wurde das Pflanzenmaterial aus der Korbpresse entfernt, aufgeschüttelt bzw. aufgelockert und in eine andere Korbpresse gefüllt. Es kann auch dieselbe Korbpresse wie zuvor verwendet werden. Dann wurde beim 2. Pressen das erhaltene Pflanzenmaterial bei etwa 300 kPa für etwa 20 min gepresst.

Die Ausbeute nach dem Pressen betrug zwischen 40 und 45% Pflanzensaft.

### In-Prozess-Kontrolle:

Eine repräsentative Probe wurde aus dem gewonnenen Pflanzensaft entnommen und untersucht. Es wurden die folgenden charakteristischen Werte bestimmt:

| | |
|---|---|
| Trockenrest: | nicht weniger als 2% (Gew./Gew.) |
| Relative Dichte (25°C): | zwischen 1000 und 1100 kg/m³ |
| pH-Wert: | 4,5 - 5,0 |

### Filtration:

Der erhaltene Pflanzensaft wurde durch eine Filterkaskade gepumpt. Die Filterkaskade bestand aus 3 Filtereinheiten: einem ersten Beutelfilter mit einer Maschenweite von 150 µm, einem zweiten Beutelfilter mit einer Maschenweite von 10 µm und einem Nanoceram^{®}-Filter mit einer Maschenweite von 2,5 µm. Es können auch andere Filter verwendet werden.

### Pasteurisieren:

Der erhaltene Pflanzensaft wurde einem Pasteurisierungsverfahren unterzogen, wobei sich die folgenden Parameter als besonders zweckmäßig herausgestellt haben:

| | | |
|---|---|---|
| - | Einlasstemperatur: | 20°C |
| - | Pasteurisierungstemperatur: | 90°C |
| - | Pasteurisierungsdauer: | 40 sek |
| - | Auslasstemperatur: | 32°C |

### pH-Einstellung:

Der pasteurisierte Pflanzensaft wurde in einen Aufbewahrungstank gefüllt. Dort wurde der pH-Wert unter Zugabe von Citronensäure auf einen Bereich von 4,5 bis 5,0 eingestellt.

### Konservierung:

Ein geeignetes Konservierungsmittelsystem wurde zugegeben. Dieses bestand aus 0,5 Gew.-% Kaliumsorbat und 0,5 Gew.-% Natriumbenzoat.

Ein Test zur Überprüfung der Konservierung zeigte ein positives Ergebnis.

### Abfüllung:

Der pasteurisierte Pflanzensaft wurde in Behälter mit unterschiedlichem Fassungsvermögen (25 Liter, 50 Liter, 100 Liter oder 200 Liter) gefüllt.

### Qualitätkontrolle des Endprodukts:

Die Qualitätskontrolle des endgültigen Produkts ergab die nachfolgenden Charakteristika:

| | | |
|---|---|---|
| - | Erscheinungsbild: | gelblich bis pinkfarben, viskose klare Flüssigkeit |
| - | Geruch: | charakteristischer Geruch |
| - | Trockenrest: | nicht weniger als 2 % (Gew./Gew.) |
| - | relative Dichte (20°C): | zwischen 1000 und 1100 kg/m³ |
| - | pH-Wert: | 4,5 - 5,0 |
| - | Identifikation: | Dünnschichtchromatographie |

### Mikrobiologie:

| | | |
|---|---|---|
| - | gesamt aerobe Belastung | max 100 CFU/g |
| - | koliforme Bakterien | --- |
| - | Hefepilze und Pilze | 10 CFU/g |
| - | E. coli Bakterien | --- |

### Aufbewahrung:

Die Bedingungen bei der Aufbewahrung waren wie folgt:
- Lagerung in geschlossenen Behältern
- Temperaturkontrolle: 25 - 27°C sollten nicht überschritten werden
- 65% relative Luftfeuchtigkeit
- Ausschluss von Licht

### Versuche zur mikrobiologischen Untersuchung und Bewertung des erfindungsgemäßen Bulbine-frutescens-Pflanzensafts

Die antimikrobiellen Eigenschaften des erfindungsgemäß hergestelltem Bulbine-frutescens-Pflanzensafts wurden untersucht.

### Kinetischer Provokationstest 1

Mit Bulbine-frutescens-Pflanzensaft wurde ein kinetischer Provokationstest an verschiedenen Organismen durchgeführt. Der Test wurde gemäß dem Verfahren der American Society for Testing and Materials (ASTM), Verfahren E1174, durchgeführt. Der Test wurde in Anwesenheit von 0,1 Gew.-% Humanserum mit einer Inokulation von 10⁶ CFU/g durchgeführt. Nach sechs Stunden wurden die folgenden logarithmischen Reduktionen beobachtet:

| | |
|---|---|
| Clostridium perfringens: | nach sechs Stunden keine logeduktion. |
| Clostridium tetani: | nach sechs Stunden keine log-Reduktion. |
| Streptococcus pyogenes Gruppe A: | nach sechs Stunden eine 4-log-Reduktion. |
| Staphylococcus aureus: | nach sechs Stunden eine 6-log-Reduktion (völlige Abtötung nach 1 Stunde). |
| Escherischia coli 0157H7: | nach sechs Stunden eine 6-log-Reduktion (völlige Abtötung nach 6 Stunden). |
| Salmonella enteritidis Serotyp Panama: | nach sechs Stunden eine 6-log-Reduktion (völlige Abtötung nach 6 Stunden). |
| Candida albicans: | nach sechs Stunden eine 1-log-Reduktion. |
| Trichophyton mentagrophytes: | nach sechs Stunden eine 2-log-Reduktion. |

Für eine Anzahl von Organismen waren die Ergebnisse überraschend gut und standen in Widerspruch zu den in der Literatur veröffentlichten Ergebnissen.

### Kinetischer Provokationstest 2

In einem zweiten Test wurde der Bulbine-frutescens-Pflanzensaft einem weiteren kinetischen Provokationstest unterzogen, aber nun in Anwesenheit von 1 Gew.-% menschlicher Fäkalien. Dieser Test wurde ebenfalls gemäß dem Verfahren der American Society for Testing and Materials (ASTM), Verfahren E1174, mit verschiedenen Inokulationen durchgeführt. Es wurde eine Dauer von zwei (2) Stunden gewählt.
Escherichia, Klebsiella & Enterobacter Spezies (aerobe gram-negative Stäbchen; Entero-bacteriaceae): Inokulation 10⁵ CFU/g. Völlige Abtötung nach 2 Stunden.
Bacteroides & Fusobacterium Spezies (anaerobe gram-negative Stäbchen): Inokulation 10⁴ CFU/g. Völlige Abtötung nach 1 Stunde.
Staphylococcus & Streptococcus Spezies (aerobe gram-positive Kokken): Inokulation 10⁴ CFU/g. Völlige Abtötung nach 2 Stunden.
Peptococcus & Peptostreptococcus (anaerobe gram-positive Kokken): Inokulation 10⁴ CFU/g. Völlige Abtötung nach 1 Stunde.
Lactobacillus & Bifidobacterium (anaerobe gram-positive nicht sporenbildende Stäbchen): Inokulation 10⁴ CFU/g. Völlige Abtötung nach 1 Stunde.
Clostridium Spezies (anaerobe gram-positive sporenbildende Stäbchen): Inokulation 10² CFU/g. Nach 2 Stunden keine Abtötung. Nach 24 Stunden eine 40%ige Reduktion.
Candida albicans: Inokulation 10² CFU/g. Völlige Abtötung nach 1 Stunde.

Die Ergebnisse waren wieder äußerst überraschend, völlig unerwartet und standen in Widerspruch zu früheren, in der Literatur angegebenen Daten. Die Ergebnisse zeigten Vorteile im Hinblick auf besondere mikrobielle Anforderungen. Der Bulbine-frutescens-Pflanzensaft kann daher als funktioneller Inhaltsstoff zum Einsatz kommen.

### Kinetischer Provokationstest 3

Ein (100 % reiner) Bulbine-frutescens-Pflanzensaft ohne weitere Zusätze wurde einem weiteren kinetischen Provokationstest an verschiedenen Organismen unterzogen. Auch dieser Test wurde gemäß dem Verfahren der American Society for Testing and Materials (ASTM) mit einer Inokulation von 10⁶ CFU/g durchgeführt. Nach 6 Stunden wurden die folgenden logarithmischen Reduktionen beobachtet:

| | |
|---|---|
| Staphylococcus aureus: | nach sechs Stunden eine 6-log-Reduktion (völlige Abtötung nach 1 Stunde). |
| Enterococcus faecium: | nach sechs Stunden eine 6-log-Reduktion (völlige Abtötung nach 1 Stunde). |
| Escherichia coli: | nach sechs Stunden eine 6-log-Reduktion (völlige Abtötung nach 1 Stunde). |
| Candida albicans: | nach sechs Stunden eine 6-log-Reduktion (völlige Abtötung nach 1 Stunde). |
| Trichophyton mentagrophytes: | nach sechs Stunden eine 6-log-Reduktion (völlige Abtötung nach 1 Stunde). |

### Versuche zu den immunstimulierenden Eigenschaften des erfindungsgemäßen Bulbine-frutescens-Pflanzensafts

### Wundheilung bei immungeschwächten Personen

Es wurden Versuche durchgeführt, um den Bulbine-frutescens-Pflanzensaft bei der Wundheilung immungeschwächter Personen, insbesondere bei Patienten mit Diabetes Typ II und schlechter Wundheilung an den unteren Extremitäten (Füßen) zu testen.

Hierzu wurden acht Freiwillige mit nicht heilenden Wunden von durchschnittlich 11 cm² ausgewählt. Vier Freiwilligen wurde ein Placebo verabreicht und vier Freiwilligen wurde Bulbine-frutescens-Pflanzensaft auf die Wunden aufgetragen. Bei den Freiwilligen, die mit Bulbine-frutescens-Pflanzensaft behandelt wurden, wurde eine deutliche Verbesserung beobachtet: die Wunden schlossen sich innerhalb von 3 bis 4 Tagen. Bei den Freiwilligen, die mit dem Placebopräparat behandelt wurden, konnte keinerlei Verbesserung festgestellt werden. Da die Wunden bereits seit einem langen Zeitraum bestanden, in einem Fall mehr als ein Jahr, konnte die Bildung einer verfärbten Narbe nicht vermieden werden.

Der Bulbine-frutescens-Pflanzensaft kann bei der Wundheilung schlecht heilender Wunden, wie etwa bei Patienten mit Diabetes Typ II, erfolgreich eingesetzt werden.

### Vorbeugende Wirkung bei Brandwunden

Es wurden Versuche durchgeführt, um den Bulbine-frutescens-Pflanzensaft bei Brandwunden zu testen, die bei Krebspatienten durch die Bestrahlung mit γ-Strahlen entstanden sind. Die Bestrahlung mit γ-Strahlen wird nach dem chirurgischen Eingriff oft präventiv angewandt, zum Beispiel nach einer partiellen oder kompletten Brustentfernung. Sechs Patientinnen mit Brustkrebs wurden gebeten, den Pflanzensaft präventiv auf den Körperbereich aufzubringen, der mit γ-Strahlen bestrahlt werden sollte. Nach dem Bestrahlungsprogramm wurden keine sichtbaren Brandwunden beobachtet, während die Kontrollgruppe mittlere bis schwere Brandwunden bekam.

Der Bulbine-frutescens-Pflanzensaft kann daher in vorteilhafter Weise eingesetzt werden, um die während der Bestrahlung mit γ-Strahlen entstehenden Brandwunden zu verhindern und/oder zu heilen.

### Versuche zur Anwendungen des erfindungsgemäßen Bulbine-frutescens-Pflanzensafts in der Kosmetik und zur Hautbehandlung

### Anwendung des Bulbine-frutescens-Pflanzensafts in Deodorants

Wie oben bereits beschrieben wurde der erfindungsgemäße Pflanzensaft gewonnen. Der Pflanzensaft wurde dann in eine deodorisierend wirkende Formulierung einbezogen. Ein Beispiel für eine derartige Formulierung ist nachfolgend angegeben:

| | |
|---|---|
| demineralisiertes Wasser | 82,7 Gew.-% |
| Stearylglucosid (Emulgator) | 4,2 Gew.-% |
| Bulbine-frutescens-Pflanzensaft | 6,0 Gew.-% |
| PPG-15-Stearylether | 4,0 Gew.-% |
| Decyloleat | 1,5 Gew.-% |
| Glycerylcaprylat | 1,2 Gew.-% |
| Hydroxyethylcellulose | 0,4 Gew.-% |
| | |
| Gesamt | 100 Gew.-% |

Der pH-Wert der Formulierung lag bei 4,5 bis 6,0.

Die gewonnene Formulierung war leicht viskos und konnte beispielsweise mit einem Roll-on-System aufgetragen werden. In einem Versuch (nicht-optimiert) wurde eine Geruchskontrolle für die Dauer von 8 Stunden durchgeführt, die in Form von "Schnüffeltests" stattfand. Die Formulierung verblieb wegen der getroffenen Auswahl von Weichmachern auf der Haut. Selbstverständlich sind auch andere beliebige Verabreichungsformen möglich, mit denen die Formulierung auf die Haut aufgebracht werden kann, wie beispielsweise in Form einer Creme, Lotion, Spray, Aerosol oder in anderer Form.

Der Bulbine-frutescens-Pflanzensaft war für den Achsel- und Genitalbereich besonders wirksam. Eine Wirksamkeit im Fall der Trimethylaminurie-Störung liegt jedoch nicht vor.

### Anwendung von erfindungsgemäßem Bulbine-frutescens-Pflanzensaft zur Behandlung von Nagelpilz und Fußpilz

Der erfindungsgemäß hergestellte Pflanzensaft wurde in eine Formulierung einbezogen. Es wurde die folgende Zusammensetzung getestet:

| | |
|---|---|
| demineralisiertes Wasser | 82,6 Gew.-% |
| Bulbine-frutescens-Pflanzensaft | 6,0 Gew.-% |
| Pentylenglycol | 5,0 Gew.-% |
| ungesättigtes Phosphatidylcholin | 4,0 Gew.-% |
| Isononylisononanoat | 2,0 Gew.-% |
| Hydroxyethylcellulose | 0,4 Gew.-% |
| | |
| Gesamt | 100 Gew.-% |

Der pH-Wert der Formulierung lag bei 4,5 bis 6,0.

Die Zusammensetzung wurde auf die Füße von Trägern mit Fußpilz-Erkrankung aufgetragen (sechs Freiwillige). Es zeigte sich, dass die Hefepilze nach 35 Tagen vollständig eliminiert waren und nach drei Monaten kein Rückfall zu verzeichnen war. Die kommensale Mikroflora auf der Haut wurde durch die Formulierung nicht nachteilig beeinflusst.

Folglich wurde gezeigt, dass der Bulbine-frutescens-Pflanzensaft bei der Behandlung von Nagel- und Fußpilz wirksam verwendet werden kann.

### Anwendung von Bulbine-frutescens-Pflanzensaft in Mundpflegeprodukten

Placebo-kontrollierte Versuche wurden durchgeführt, in denen sich zeigte, dass das Ausspülen der Mundhöhle zweimal täglich mit 10%iger wässriger Lösung des Bulbine-frutescens-Pflanzensafts unerwünschten Mundgeruch vollständig eliminierte. Gleichzeitig wurde beobachtet, dass kleine Infektionen des Zahnfleisches, die z. B. durch Streptococcus mutans und/oder S. sanguis verursacht wurden, reguliert werden konnten. Entzündungen des Zahnfleisches und der Mundschleimhaut, die mit 10%iger wässriger Bulbine-frutescens-Pflanzensaft-Lösung behandelt wurden, verschwanden für gewöhnlich in 1 bis 2 Tagen, womit sie herkömmliche pharmazeutische Präparate, einschließlich der auf Chlorhexidindiglukonat und Wasserstoffperoxid basierenden Präparate zur Munddesinfektion, übertrafen. S. salivarius wurde vom Bulbine-frutescens-Pflanzensaft kaum beeinträchtigt. Demgegenüber sind Chlorhexidin und Wasserstoffperoxid für die kommensale Mikroflora in der Mundhöhle stark schädigend.

Der Bulbine-frutescens-Pflanzensaft war für die Mundpflege in hohem Maße wirksam, es ist sogar handelsüblichen Produkten überlegen.

### Anwendung von Bulbine-frutescens-Pflanzensaft in Anti-Ageing-Produkten

Eine Creme, enthaltend den erfindungsgemäßen Pflanzensaft, mit folgender Zusammensetzung wurde auf ihre Anti-Aging-Eigenschaften getestet:

| | |
|---|---|
| demineralisiertes Wasser | 69,0 Gew.-% |
| Ethyhexylstearat | 6,0 Gew.-% |
| Cetearylalkohol | 6,0 Gew.-% |
| Bulbine-frutescens-Pflanzensaft | 6,0 Gew.-% |
| Pentylenglycol | 5,0 Gew.-% |
| Glycerin | 3,0 Gew.-% |
| Ceteareth-6 | 2,5 Gew.-% |
| Ceteareth-25 | 2,5 Gew.-% |
| | |
| Gesamt | 100 Gew.-% |

Der pH-Wert der Creme lag bei 5,0 bis 6,0.

Als deutliche Anti-Aging-Eigenschaften wurden die Hautelastizität, die Hautfestigkeit, die Werte der Corneometer-Messung und der trans-epidermale Wasserverlust (TEWL) sowie die Zelldifferenzierung und -proliferation herangezogen. Die Ausrüstung wurde von der Courage & Khazaka Electronic GmbH, Köln, Deutschland, bezogen. Es zeigte sich, dass eine zweimalige tägliche Anwendung der Creme zu einer um 40 % verbesserten Elastizität und zu einer um 24 % verbesserten (Zug-)Festigkeit der Haut führte, wobei der Wasserverlust des subkutanen Gewebes an die Umgebung im Verhältnis zum Standard stark verringert wurde.

Weitere Versuche zeigten, dass der Zellstoffwechsel um 15 % anstieg. Eine Sichtprüfung der Haut zeigte ein deutlich verbessertes Hautbild.

### Anwendung von Bulbine-frutescens-Pflanzensaft bei topischer Kandidose

Getestet wurde eine Creme, enthaltend

| | |
|---|---|
| demineralisiertes Wasser | 69,0 Gew.-% |
| Ethyhexylstearat | 6,0 Gew.-% |
| Cetearylalkohol | 6,0 Gew.-% |
| Bulbine-frutescens-Pflanzensaft | 6,0 Gew.-% |
| Pentylenglycol | 5,0 Gew.-% |
| Glycerin | 3,0 Gew.-% |
| Ceteareth-6 | 2,5 Gew.-% |
| Ceteareth-25 | 2,5 Gew.-% |
| | |
| Gesamt | 100 Gew.-% |

auf ihre Wirkung gegen opportunistische Candida albicans auf der Haut von sechs Freiwilligen. Der pH-Wert der Creme lag bei 5,0 bis 6,0. Obwohl die topische Kandidose mit Antibiotika gewöhnlich schwer zu behandeln ist, zeigte sich, dass die Creme wirksam war, selbst nach der Behandlung anderer Erkrankungen mit Antibiotika, die dem Candida albicans oft die Chance gibt, opportunistisch zu werden. Die Behandlung mit der Creme führte für gewöhnlich nach einer 3- bis 7-tägigen Behandlung zum Verschwinden der roten Punkte auf der Haut.

Es wird somit erfindungsgemäß erstmals Bulbine-frutescens-Pflanzensaft bereitgestellt, dessen Wirkstoff auf pflanzlicher Basis ist, der sowohl im therapeutischen als auch kosmetischen Bereich für die Anwendung auf der Haut und Schleimhaut zum Einsatz kommen kann, in einer Vielzahl von Darreichungsformen formuliert werden kann und ein breites Wirkungsspektrum bietet. Die Wirksamkeit ist zum Teil überraschend hoch, wobei handelsübliche Produkte übertroffen werden. Trotzdem liegt eine hohe Verträglichkeit bei der Verwendung vor, wobei gleichzeitig die Eigenschaften der Haut- und Schleimhaut positiv beeinflusst werden.

## Patentansprüche

1. Bulbine-frutescens-Pflanzensaft, erhältlich durch die Schritte:
(1) Waschen der Blätter der Bulbine frutescens mit Wasser und gegebenenfalls Zerkleinern der Blätter;
(2) Auspressen der Blätter unter Erhalt eines Presskuchens und von Pflanzensaft;
(3) Auflockern des erhaltenen Presskuchens;
(4) Auspressen des aufgelockerten Presskuchens unter Erhalt von weiterem Pflanzensaft;
(5) Zusammengeben des Pflanzensafts aus Schritt (2) und (4) und
(6) Pasteurisieren des aus Schritt (5) erhaltenen Pflanzensafts.

2. Pflanzensaft nach Anspruch 1,
**dadurch gekennzeichnet,**
**dass** das Auspressen gemäß den Verfahrensschritten (2) und (4) bei einem Druck im Bereich von etwa 200 bis 400 kPa, bevorzugt etwa 250 bis 350 kPa, noch bevorzugter 270 bis 320 kPa, insbesondere bei etwa 300 kPa, bevorzugt für eine Dauer von einigen Minuten bis etwa 2 Stunden, besonders bevorzugt etwa 10 Minuten bis etwa 1,5 Stunden, noch bevorzugter etwa 30 Minuten bis etwa 1 Stunde durchgeführt wird.

3. Pflanzensaft nach Anspruch 1 oder 2,
**dadurch gekennzeichnet,**
**dass** das Auspressen gemäß Verfahrensschritt (2) in einem dreistufigen Press-Verfahren durchgeführt wird, umfassend
(I) Pressen der Blätter bei einem Druck im Bereich von etwa 10 bis 100 kPa, bevorzugt etwa 20 bis 80 kPa, bevorzugter etwa 30 bis 70 kPa, insbesondere etwa 50 kPa für eine vorbestimmte Zeitdauer;
(II) Nachfüllen der aus Verfahrensschritt (1) erhaltenen gewaschenen, gegebenenfalls zerkleinerten Blätter und dann Pressen bei einem Druck im Bereich von etwa 50 bis 200 kPa, bevorzugter etwa 70 bis 180 kPa, noch bevorzugter 80 bis 150 kPa, insbesondere etwa 100 kPa für eine vorbestimmte Zeitdauer und
(III) Nachfüllen der aus Verfahrensschritt (1) erhaltenen gewaschenen, gegebenenfalls zerkleinerten Blätter und dann Pressen bei einem Druck im Bereich von etwa 200 bis 400 kPa, bevorzugter etwa 250 bis 350 kPa, noch bevorzugter 270 bis 320 kPa, insbesondere etwa 300 kPa für eine vorbestimmte Zeitdauer.

4. Pflanzensaft nach Anspruch 3,
**dadurch gekennzeichnet,**
**dass** die vorbestimmte Zeitdauer im dreistufigen Press-Verfahren jeweils im Bereich von einigen Minuten bis 2 Stunden, bevorzugter etwa 5 Minuten bis 1,5 Stunden, noch bevorzugter etwa 10 Minuten bis etwa 1 Stunde, ganz besonders bevorzugt etwa 10 Minuten bis 30 Minuten eingestellt wird.

5. Pflanzensaft nach mindestens einem der vorangehenden Ansprüche 1 bis 4,
**dadurch gekennzeichnet,**
**dass** der aus Verfahrensschritt (5) erhaltene Pflanzensaft vor dem Pasteurisieren filtriert wird.

6. Pflanzensaft nach mindestens einem der vorangehenden Ansprüche 1 bis 5,
**dadurch gekennzeichnet,**
**dass** nach Durchführen des Verfahrensschritts (6) der pH-Wert des erhaltenen Pflanzensafts unter Verwendung einer pharmazeutisch oder kosmetisch akzeptablen Säure im Bereich von etwa 3,0 bis 6,0, bevorzugter etwa 3,5 bis 5,5, noch bevorzugter etwa 4,0 bis 5,5, insbesondere etwa 4,5 bis 5,0 eingestellt wird.

7. Pflanzensaft nach Anspruch 6,
**dadurch gekennzeichnet,**
**dass** die pharmazeutisch oder kosmetisch akzeptable Säure ausgewählt wird aus einer organischen Säure, bevorzugt ausgewählt aus der Gruppe, bestehend aus Adipinsäure, Alginsäure, Ascorbinsäure, Isoascorbinsäure (Erythorbinsäure), Asparaginsäure, Benzolsulfonsäure, Bernsteinsäure, 2-Acetoxybenzoesäure, Camphersäure, Camphersulfonsäure, Zimtsäure, Citronensäure, insbesondere Citronensäure-Monohydrat, Digluconsäure, Ethansulfonsäure, Glutaminsäure, Glykolsäure, Heptansäure, Hexansäure, Fumarsäure, 2-Hydroxyethansulfonsäure (Isethionsäure), Milchsäure, Maleinsäure, Hydroxymaleinsäure, Äpfelsäure, Malonsäure, Mandelsäure, Pamoasäure, Pectinsäure, Phenylessigsäure, 3-Phenylpropionsäure, Picrinsäure, Pivalinsäure, Pyruvinsäure, Stearinsäure, Sulfanilsäure, Weinsäure, p-Toluolsulfonsäure oder Mischungen dieser, bevorzugt Citronensäure, insbesondere Citronensäure-Monohydrat.

8. Pflanzensaft nach mindestens einem der vorangehenden Ansprüche 1 bis 7,
**dadurch gekennzeichnet,**
**dass** das Pasteurisieren bei einer Temperatur im Bereich von etwa 50 bis 100°C, bevorzugter 60 bis 90°C, noch bevorzugter 70 bis 90°C,
bevorzugt für eine Dauer von wenigen Sekunden bis Minuten, bevorzugter etwa 10 Sekunden bis 2 Minuten, noch bevorzugter weniger als 1 Minute, insbesondere weniger als 45 Sekunden durchgeführt wird.

9. Pflanzensaft nach mindestens einem der vorangehenden Ansprüche 1 bis 8,
**dadurch gekennzeichnet,**
**dass** ein Konservierungsmittel zugesetzt wird, ausgewählt aus der Gruppe, bestehend aus Benzoesäure, Sorbinsäure, Parabenen (Methyl-, Ethyl-, Propyl-, Butyl-, Isobutyl- oder Benzyl-Paraben), Ameisensäure, Essigsäure, Propionsäure, Salicylsäure, p-Anissäure, Lävulinsäure und deren wasserlöslichen Salzen, Glycerol-/Polyglycerolmonoestern von Caprylsäure, Caprinsäure-Laurinestern, Diolen, wie 1,2-Dihydroxypentan, 1,2-Dihydroxyhexan oder 1,2-Dihydroxyoctan, oder 1-(2-Ethylhexyl)-Glycerylether oder Mischungen dieser.

10. Verfahren zur Herstellung von Bulbine-frutescens-Pflanzensaft nach einem der vorangehenden Ansprüche 1 bis 9, umfassend die Schritte:
(1) Waschen der Blätter der Bulbine frutescens mit Wasser und gegebenenfalls Zerkleinern der Blätter;
(2) Auspressen der Blätter unter Erhalt eines Presskuchens und von Pflanzensaft;
(3) Auflockern des erhaltenen Presskuchens;
(4) Auspressen des aufgelockerten Presskuchens unter Erhalt von weiterem Pflanzensaft;
(5) Zusammengeben des Pflanzensafts aus Schritt (2) und (4) und
(6) Pasteurisieren des aus Schritt (5) erhaltenen Pflanzensafts.

11. Verfahren nach Anspruch 10,
**dadurch gekennzeichnet,**
**dass** der erhaltene Pflanzensaft einer Weiterverarbeitung unterzogen wird, aus ein oder mehreren der nachfolgenden Verfahrensweisen, die in beliebiger Reihenfolge kombiniert werden können: Filtrieren, Wasserverdampfung unter vermindertem Druck und/oder erhöhter Temperatur, azeotrope Destillation unter vermindertem Druck und/oder erhöhter Temperatur unter Verwendung eines geeigneten Co-Lösemittels, Sprühtrocknen oder Gefriertrocknen.

12. Bulbine-frutescens-Pflanzensaft nach mindestens einem der vorangehenden Ansprüche 1 bis 9 zur Verwendung im therapeutischen Bereich, bevorzugt zur Verwendung in der Behandlung von Haut- oder Schleimhauterkrankungen, insbesondere Infektionen oder Entzündungen der Haut oder Schleimhaut, wie Pilzerkrankungen, oder in der Behandlung oder Vorbeugung von Verletzungen der Haut oder Schleimhaut, insbesondere bei (Brand-)Wunden.

13. Bulbine-frutescens-Pflanzensaft nach mindestens einem der vorangehenden Ansprüche 1 bis 9 zur Verwendung als Desinfektionsmittel, insbesondere gegen Staphylococcus aureus, einschließlich Methicillin-resistentem Staphylococcus aureus (MRSA) und Clindamycin-, Erythromycin- und Ciprofloxacin-resistentem Staphylococcus aureus (CEC-SA), oder als Mittel zur Stärkung der Immunabwehr.

14. Bulbine-frutescens-Pflanzensaft nach mindestens einem der vorangehenden Ansprüche 1 bis 9 zur Verwendung im kosmetischen Bereich, bevorzugt zur Körperpflege, Mundpflege, in Deodorants und als Anti-Ageing-Produkt.

15. Pharmazeutische Zusammensetzung, umfassend den Bulbine-frutescens-Pflanzensaft nach mindestens einem der vorangehenden Ansprüche 1 bis 9, in Mischung mit einem oder mehreren pharmazeutisch oder kosmetisch akzeptablen Trägermedien oder Hilfsstoffen.

16. Kosmetische Zusammensetzung, umfassend den Bulbine-frutescens-Pflanzensaft nach mindestens einem der vorangehenden Ansprüche 1 bis 9, in Mischung mit einem oder mehreren pharmazeutisch oder kosmetisch akzeptablen Trägermedien oder Hilfsstoffen.
